# EUROPEAN PATENT APPLICATION

(11) **EP 2 019 089 A1**
(43) Date of publication of application: **28.01.2009**
(21) Application number: 07707334.4
(22) Date of filing: 24.01.2007
(51) Int. Cl.: C07C 37/82, B01D 9/02, C07C 37/86, C07C 39/16, C07B 61/00

(54) **PROCESS FOR PRODUCING HIGH-PURITY BISPHENOL A AND PRODUCTION APPARATUS**

(30) Priority: 17.05.2006 JP 2006138103
(71) Applicant: Idemitsu Kosan Co., Ltd., Chiyoda-ku Tokyo 100-8321 (JP)
(72) Inventor: YOSHITOMI, Kazuyuki, Chiba 299-0193 (JP); KODAMA, Masahiro, Chiba 299-0193 (JP); MASUDA, Shuichi, Chiba 299-0193 (JP); KOHIRUIMAKI, Jun, Chiba 299-0193 (JP); YAMASAKI, Hokuto, Chiba 299-0193 (JP)
(74) Representative: Vossius & Partner
(86) International application number: PCT/JP2007/051087
(87) International publication number: WO 2007/132575

(57) **Abstract**

A process for producing bisphenol A containing a free-acid removing step in the following step (D) so as to remove free acid contained in a mother liquid or an isomerized liquid, in a process for producing bisphenol A including: a condensation reaction step (A) reacting phenol with acetone in the presence of an acidic catalyst, a concentration step (B) concentrating the reaction mixture, a crystallization and solid-liquid separation step (C) separating into an adduct of bisphenol A and phenol and a mother liquid by cooling the concentrated reaction mixture, an isomerization step (D) isomerizing the whole of the mother liquid with an isomerization catalyst and circulating the resultant isomerized liquid to the step (A) and/or step (B), as necessary, a recovery step (E) recovering an adduct of bisphenol A and phenol from part of the isomerized liquid treated in the step (D), an adduct decomposition step (F) preparing a bisphenol A melt by removing phenol from the adduct obtained in the step (C), and a prilling step (G) preparing product prill by granulating the bisphenol A melt.

## Description

### Technical Field

The present invention relates to, in an isomerization step that is one of the processes for producing bisphenol A, a process for producing high-quality bisphenol A with an excellent transparency (color) in particular by removing trace amount of free acid that is eluted off an acidic catalyst used in a reaction step and an isomerization catalyst used in an isomerization step in which the whole mother liquid obtained in a crystallization and solid-liquid separation step is isomerized, and to an apparatus for producing the bisphenol A.

### Background Art

In recent years, polycarbonate that has had a growing demand as an engineering plastic, particularly has had a rapidly growing demand as an optical material for optical disc substrates and the like.
Polycarbonate used as an optical material is required to be infinitely transparent (low color). Naturally, bisphenol A that is a raw material of polycarbonate is also required to be the same.
Bisphenol A is usually produced by reacting acetone and excess amount of phenol in the presence of an acidic catalyst. The acidic catalyst includes typically a strong acid cation exchange resin. The reaction mixture contains, besides bisphenol A, unreacted phenol, unreacted acetone, and a by-product such as reaction product water, a color substance and the like. The reaction mixture is usually separated into a mother liquid and crystals by crystallization and solid-liquid separation after the unreacted acetone and reaction product water are removed by evaporation and purified. Here, a production process (production method 1) has been known, in which the resultant mother liquid is returned to the reaction step as a raw material because the mother liquid contains a large amount of phenol. Another production method (production method 2) has been also known, in which after the unreacted acetone and reaction product water are removed by evaporation, while evaporated phenol is returned to the reaction step, the mother liquid obtained in the crystallization and solid-liquid separation step is isomerized with an acidic catalyst, and the resultant isomerized liquid is returned to the reaction mixture that is obtained after the unreacted acetone and reaction product water are removed by evaporation.
In either production method, trace amount of free acid that is eluted off the acidic catalyst used is concentrated and accumulated in the reaction mixture by circulating the mother liquid repeatedly. This free acid, sulfonic acid in particular, is responsible for forming color substances and bringing about color degradation of product bisphenol A, and also responsible for causing thermal decomposition.

Patent Documents 1 and 2 disclose a method for preventing thermal decomposition of bisphenol A by incorporating a step of bringing a mother liquid, which is obtained by reacting phenol and acetone to form bisphenol A and removing bisphenol A in the form of an adduct of bisphenol A and phenol from the resultant reaction product liquid, into contact with a basic ion exchange resin.
However, in the production method 2 where the mother liquid is isomerized with an acidic catalyst, this method hardly removes the free acid that is eluted off the acidic catalyst used for isomerization.
Further, in the production step 1, Patent Document 3 discloses a method for producing a crystalline adduct of bisphenol A and phenol, which is used for producing high-quality bisphenol A, by incorporating a free-acid removing step using a weak base ion exchange resin at an inlet and an outlet of the reaction step and in a circulation line circulating the mother liquid separated in the crystallization step to the reaction step and by removing a free-acid eluted off an acidic catalyst used in a reaction step.
Patent Document 4 discloses a method for producing bisphenol A, in a reaction step (A) of forming bisphenol A by reacting phenol and acetone, by bringing at least part of phenolic compounds into contact with a strong acid cation exchange resin and a strong base anion exchange resin before the phenolic compounds are used, and/or bringing at least part of the reaction mixture and/or at least part of the mother liquid into contact with a strong acid cation exchange resin and a strong base anion exchange resin before the mother liquid is returned to the reaction mixture.
However, these Patent Documents 1 to 4 do not describe at all about the production method 2 that includes an isomerization step and do not also disclose at all a method for incorporating the free-acid removing step in the isomerization step.

Patent Document 1: Japanese Patent Laid-Open Publication No. H01-156937,
Patent Document 2: Japanese Patent Laid-Open Publication No. H05-201905,
Patent Document 3: Japanese Patent Laid-Open Publication No. 2001-306813,
Patent Document 4: Japanese Patent Laid-Open Publication No. H06-9468.

### Disclosure of the Invention

### Problems to be Solved by the Invention

It is an object of the present invention to provide a process for producing high-quality bisphenol A with an excellent transparency (color) in particular, and an apparatus for producing the bisphenol A.

### Means for Solving the Problems

The present inventors have made intensive studies to achieve the aforementioned object. As a result, it has been found that high-quality bisphenol A with an excellent transparency (color) in particular is produced by incorporating a free-acid removing step in an isomerization step so as to remove free acid contained in a mother liquid or isomerization liquid. The present invention is accomplished based on this finding.

Namely, the present invention provides:
(1) In a process for producing bisphenol A including: a condensation reaction step (A) reacting excess amount of phenol with acetone in the presence of an acidic catalyst, a concentration step (B) concentrating a reaction mixture obtained in the step (A), a crystallization and solid-liquid separation step (C) crystallizing an adduct of bisphenol A and phenol by cooling the concentrated reaction mixture obtained in the step (B) and separating into the adduct and a mother liquid, an isomerization step (D) isomerizing the whole of the mother liquid obtained in the step (C) with an isomerization catalyst and isomerizing the resultant isomerized liquid back to the step (A) and/or step (B), an adduct decomposition step (F) preparing a bisphenol A melt by removing phenol from an adduct of bisphenol A and phenol obtained in the step (C), and a prilling step (G) preparing product prill by granulating the bisphenol A melt obtained in the step (F), a process for producing bisphenol A containing a free-acid removing step in the step (D) so as to remove free acid contained in the mother liquid or the isomerized liquid;
(2) In a process for producing bisphenol A including: a condensation reaction step (A) reacting excess amount of phenol with acetone in the presence of an acidic catalyst, a concentration step (B) concentrating a reaction mixture obtained in the step (A), a crystallization and solid-liquid separation step (C) crystallizing an adduct of bisphenol A and phenol by cooling the concentrated reaction mixture obtained in the step (B) and separating into the adduct and a mother liquid, an isomerization step (D) isomerizing the whole of the mother liquid obtained in the step (C) with an isomerization catalyst and returning part of the resultant isomerized liquid to the step (B), a recovery step (E) recovering an adduct of bisphenol A and phenol from a remaining portion of the isomerized liquid treated in the step (D), an adduct decomposition step (F) preparing a bisphenol A melt by removing phenol from an adduct of bisphenol A and phenol obtained in the step (C), and a prilling step (G) preparing product prill by granulating the bisphenol A melt obtained in the step (F), a process for producing bisphenol A containing a free-acid removing step in the step (D) so as to remove free acid contained in the mother liquid or the isomerized liquid;
(3) The process for producing bisphenol A as described in (1) or (2), wherein the mother liquid before the isomerization is treated in the free-acid removing step so as to remove free acid;
(4) The process for producing bisphenol A as described in (1) or (2), wherein the isomerized liquid isomerized by passing the mother liquid through the isomerization catalyst is treated in the free-acid removing step so as to remove free acid;
(5) The process for producing bisphenol A as described in any one of (1) to (4), wherein free acid is removed by using an anion exchange resin in the free-acid removing step;
(6) The process for producing bisphenol A as described in any one of (1) to (4), wherein free acid is removed by using a mixture of an anion exchange resin and a cation exchange resin in the free-acid removing step;
(7) The process for producing bisphenol A as described in (5) or (6), wherein the anion exchange resin is a weak base anion exchange resin;
(8) The process for producing bisphenol A as described in (6), wherein the cation exchange resin is a strong acid cation exchange resin;
(9) The process for producing bisphenol A as described in any one of (1) to (8), wherein free acid is removed in the presence of 0.01 to 1 % by mass of water in the free-acid removing step; and
(10) An apparatus for producing bisphenol A equipped with a free-acid removal measures to remove free acid contained in a mother liquid or an isomerized liquid in the isomerization step (D) in the process for producing bisphenol A as described in (1) or (2).

### Brief Description of the Drawings

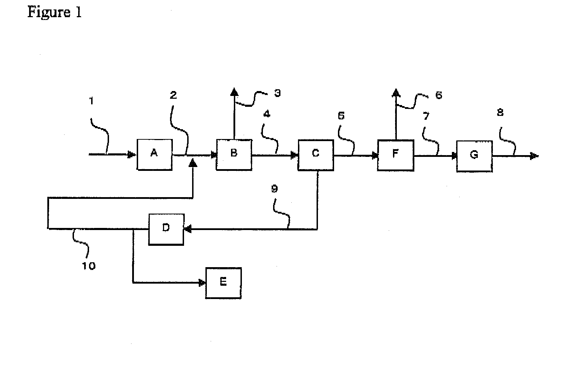

FIG. 1 shows an example of a flow sheet upon practicing the present invention.

### Explanation of Reference Numerals

- A:: Condensation reaction step,
- B:: Concentration step,
- C:: Crystallization and solid-liquid separation step,
- D:: Isomerization and free-acid removing step,
- E:: Recovery step,
- F:: Adduct decomposition step,
- G:: Trilling step,
- 1:: Acetone and phenol (raw material),
- 2:: Reaction mixture,
- 3:: Unreacted acetone, reaction product water, phenol, and the like,
- 4:: Concentrated liquid,
- 5:: Crystalline adduct,
- 6:: Phenol,
- 7:: Bisphenol A melt,
- 8:: Product bisphenol A prill,
- 9:: Mother liquid, and
- 10:: Isomerized liquid.

### Best Mode for Carrying out the Invention

A process for producing bisphenol A according to the present invention is characterized in that trace amount of free acid eluted off an acidic catalyst used in a reaction step and an isomerization catalyst used in an isomerization step is removed by incorporating a free-acid removing step at either side of up-flow or down-flow of the isomerization step.

A process for producing bisphenol A according to the present invention includes,
(A) a condensation reaction step, in which acetone and excess amount of phenol are reacted in the presence of an acidic catalyst; (B) a concentration step, in which the reaction mixture obtained in the condensation reaction step (A) is concentrated; (C) a crystallization and solid-liquid separation step, in which the concentrated reaction mixture obtained in the concentration step (B) is cooled so as to crystallize an adduct of bisphenol A and phenol and is separated into a mother liquid and the adduct; (D) an isomerization step having a free-acid removing step, in which the whole of the mother liquid obtained in the crystallization and solid-liquid separation step (C) is isomerized with an isomerization catalyst; as necessary, (E) a recovery step, in which a bisphenol A is recovered from part of the remaining portion of the isomerized liquid treated in the isomerization step (D); (F) an adduct decomposition step, in which phenol is removed from the adduct of bisphenol A and phenol obtained in the crystallization and solid-liquid separation step (C) so as to obtain a bisphenol A melt; (G) a prilling step, in which the bisphenol A melt obtained in the adduct decomposition step (F) is granulated into product prill; and the like.

Each step will be mentioned in detail below.

### (A) Condensation reaction step

Source phenol and acetone are reacted in a manner that phenol is in excess stoichiometrically. The molar ratio of phenol/acetone is usually in the range from 3 to 30 and preferably from 5 to 20. The reaction temperature is usually from 50 to 100°C. The reaction pressure is usually from normal pressure to 1.5 MPa and preferably from normal pressure to 0.6 MPa. As a catalyst, a strong acid cation exchange resin such as a sulfonic acid ion-exchange resin and the like is usually used. In addition, a catalyst that is obtained by neutralizing part of the strong acid cation exchange resin catalyst with an auxiliary catalyst such as mercapto alkylamine and the like may be also used. The catalyst may include, for example, a catalyst in which 5 to 30 mol% of sulfonic acid group is neutralized with 2-mercaptoethylamine, 3-mercaptopropylamine, N,N-dimethyl-3-mereaptopropylamine, N,N-di-n-butyl-4-mer-captobutylamine, 2,2-dimethylthiazolidine, and the like.

The source liquid of phenol and acetone is reacted in a fixed-bed flow process that is a continuous process and also a plug-flow process or in a suspension-bed batch process. In the case of the fixed-bed flow process, the liquid hourly space velocity (LHSV) of the source liquid supplied to a reactor is from about 0.2 to 50 hr⁻¹. In the case of the suspension-bed batch-wise process, the amount of a resin catalyst is in the range of generally from 20 to 100 % by mass with respect tn the source liquid and the reaction time is about 0.5 to 5 hours, although they depend on the reaction temperature and pressure.

### (B) Concentration step

The reaction mixture from the condensation reaction step is usually concentrated in two steps. In a first concentration step, unreacted acetone, reaction product water, and the like are removed by vacuum distillation or the like. Vacuum distillation is performed at a temperature of about 30 to 180°C under a pressure of about 13 to 67 kPa. Subsequently, in a second concentration step, phenol is distilled out and the bisphenol A concentration is adjusted. In this occasion, the bisphenol A concentration is preferably adjusted at about 20 to 60 % by mass. When the bisphenol A concentration is lower than 20 % by mass, the yield becomes low. At a bisphenol A concentration of higher than 60 % by mass, the solidification temperature becomes high, this results in easy solidification, and a problem in which the reaction mixture is not transported practically may be caused. Therefore, the bisphenol A concentration is usually adjusted by preliminary concentrating the reaction mixture in the first concentration step. Preferably, the second concentration step is usually performed under a pressure of about 4 to 40 kPa and at a temperature of about 70 to 140°C.

### (C) Crystallization and solid/liquid separation step

The concentrated liquid from the concentration step is usually cooled around 70 to 140°C to around 35 to 60°C so as to crystallize an adduct (crystalline adduct) of bisphenol A and phenol and to obtain the liquid in a slurry state. The concentrated liquid is cooled by removing heat by way of an external heat-exchanger or by using vaporization latent heat of water that is added to a crystallizer. After that, the liquid in a slurry state is separated into solid and liquid. The composition of a mother liquid obtained in this crystallization and solid-liquid separation step is usually as follows: phenol is from 65 to 85 % by mass, bisphenol A is from 10 to 20 % by mass, and a by-product such as a 2,4'-isomer and the like is from 5 to 15 % by mass. The mother liquid contains a large amount of an impurity such as a 2,4'-isomer and the like. The whole of the mother liquid is treated in a subsequent isomerization step so as to convert the 2,4'-isomer into bisphenol A and to recover phenol and bisphenol A contained in the mother liquid.
The crystalline adduct of bisphenol A and phenol separated by solid-liquid separation of the reaction mixture slurry is fed to an adduct decomposition step in which phenol is removed so as to obtain high purity bisphenol A.

A solid containing as a main component the crystalline adduct that is filtered and deposited on the surface of a filter of a solid-liquid separator is subjected to washing with a washing liquid. The washing liquid may include phenol that is recovered by evaporation, source phenol, water, a water-phenol mixed liquid, and also may include a solution similar to a bisphenol A-saturated phenol solution. The much the amount of the washing liquid used the better of course, from the viewpoint of washing efficiency. However, the amount has an own upper limit considering the redissolution loss of crystalline adduct and the circulation, recovery and reuse of the washing liquid. The amount considered to be most efficient is usually about 0.1 to 10 times of the crystals on a mass basis.
Note that, the crystalline adduct may be redissolved after the crystallization and solid/liquid separation, and the crystallization and solid/liquid separation may be repeated. The impurities incorporated in the adduct crystals are decreased successively by repeating the crystallization and solid/liquid separation in multi-stages. In this occasion, as a dissolution liquid used for the redissolution and as a washing liquid used to wash the solid containing an adduct as a main component that is obtained by liquid-solid separation and contains mainly the adduct, phenol that is recovered by evaporation, source phenol, water, and a water-phenol mixed liquid, and also a solution similar to a bisphenol A-saturated phenol solution may be used in each stage. The mother liquid obtained by recrystallization and solid/liquid separation may be recycled to the crystallization step in the former stage.

### (D) Isomerization step

The whole of the liquid portion (mother liquid) that is obtained in the crystallization and solid-liquid separation step is supplied to an isomerization step in which by-products contained in the mother liquid are isomerized. In addition, free acid contained in the mother liquid is removed in a free-acid removing step. Most of the isomerized liquid is returned to the condensation reaction step (A) and/or the concentration step (B) and preferably to the concentration step (B). Here, if necessary, part of the isomerized liquid is taken out so as to prevent accumulation of impurities, and the taken-out liquid is fed to a recovery step as a discharge liquid.
In the isomerization, usually a sulfonic acid cation exchange resin is used as a catalyst. The reaction temperature is from about 50 to 100°C. The liquid hourly space velocity (LHSV) is from about 0.2 to 50 hr⁻¹ in the case of a fixed-bed flow process that is a continuous process and also a plug-flow process.

The free-acid removing step in the present invention may be provided on either side of up-flow or down-flow of the isomerization step.
As an ion exchange resin that is used in the free-acid removing step of the present invention, a cation exchange resin and an anion exchange resin are used. As the cation exchange resin, a strong acid ion exchange resin is used. As the anion exchange resin, a strong base ion exchange resin and a weak base ion exchange resin are used. A strong acid ion exchange resin having as a functional group a sulfonic acid group (RSO₃⁻H⁺), a weak acid cation exchange resin having as a functional group a carboxylic acid group (R-COO⁻H⁺), a phosphonic acid group (R-P(O)(O⁻H⁺)₂), a phosphinic acid group (R-PH(O)(O⁻H⁺)), an arsenious acid group (R-AsO⁻H⁺), or a phenoxide group (R-C₆H₄O⁻H⁺), and the like are known, but a sulfonic acid strong acid ion exchange resin is usually used and preferable.
The strong base anion exchange resin has a quaternary ammonium base (R-N⁺R₁R₂R₃) or a tertiary sulfonium group (R-S⁺R₁R₂) as a functional group.
As a commercially available product, LEWATIT (registered trade mark) MP-62 manufactured by Bayer Corporation and AMBERLIST (registered trade mark) A26 manufactured by R&H Corporation are included.

A weak base anion exchange resin having primary to tertiary amine as a functional group may be included. As a commercially available product, DIAION (registered trade mark) WA-20, 21, and 30 manufactured by Mitsubishi Chemical Corporation and A21 manufactured by R&H Corporation are included.
The temperature at which the strongly basic anion exchange resin is used in the free-acid removing step is preferably from 40 to 80°C. When the temperature is lower than 40°C, possibly bisphenol A is deposited in the free-acid removing step. When the temperature exceeds 80°C, elimination of the functional group occurs.
The temperature at which the weak base ion exchange resin is used is preferably from 40 to 100°C and more preferably from 60 to 80°C. When the temperature is lower than 40°C, possibly bisphenol A is deposited in the free-acid removing step. When the temperature exceeds 100°C, elimination of the functional group occurs.
Regarding the anion exchange resin used in the free-acid removing step, the anion exchange resin is brought into contact with the mother liquid in which the water concentration is adjusted at preferably 0.01 to 1 % by mass and more preferably 0.1 to 0.7 % by mass. At the water concentration of less than 0.01 % by mass, impurities contained in the mother liquid clog the micropores inside of the ion exchange resin, and the operating life of the catalyst may be shortened. On the other hand, when the water concentration is higher than 1 % by mass, water that is a polar substance activates the motion of cationic electrons, so that the collection capability of the anion exchange resin may be lowered, and the operating life of the catalyst may be shortened.
The water concentration of the mother liquid may be adjusted by adding water to the mother liquid, or by reducing the water concentration to a predetermined value through vaporization in which the water (3 to 7 % by mass) contained in the mother liquid is vaporized in the crystallization and solid-liquid separation step (C) where vacuum vaporization crystallization accompanied by water addition is applied.

There is no limitation on the mixing ratio when a cation exchange resin and an anion exchange resin are mixed and used in the free-acid removing step, but the cation exchange resin and anion exchange resin may be mixed in a volume ratio of 1:5 to 5:1.
Note that, as the anion exchange resin used in the present invention, a weak base anion exchange resin is preferable. In the free-acid removing step, in the case of a fixed-bed flow process that is a continuous process and also a plug-flow process, the liquid hourly space velocity (LHSV) is preferably from 0.02 to 20 hr⁻¹.

An apparatus for producing bisphenol A according to the present invention is
characterized by being equipped with a free acid removing measures that removes free acid contained in the mother liquid or isomerization liquid. For the free acid removing measures, an ion exchange resin similar to the ion exchange resin used in the above free-acid removing step is used. As the ion exchange resin, a cation exchange resin and an anion exchange resin are included. As the cation exchange resin, a strong acid ion exchange resin is used. As the anion exchange resin, a strong base ion exchange resin and a weak base ion exchange resin are used.

### (E) Recovery step

In the part of the isomerization liquid fed from the isomerization step, bisphenol A is contained in an amount of from about 15 to 20 % by mass and a by-product such as a 2,4'-isomer and the like is contained in an amount of from about 5 to 10 % by mass.
This isomerization liquid is concentrated, and then cooled in the presence of phenol, so that an adduct (crystalline adduct) of bisphenol A and phenol is crystallized and is subjected to solid-liquid separation. After that, the crystalline adduct is melted, and then returned to the concentration step and/or crystallization and solid-liquid separation step. After the solid-liquid separation, the resultant mother liquid is disposed after phenol is recovered.

### (F) Adduct decomposition step

In the above crystallization and solid-liquid separation step (C), the crystalline adduct recovered by solid-liquid separation is treated into high purity bisphenol A by removing phenol in an adduct decomposition step. For example, usually, the crystalline adduct is decomposed into bisphenol A and phenol by heating and melting it at about 100 to 160°C. Most of phenol is removed from the resultant melt with the use of an evaporator or the like. Further, the remaining phenol is removed by steam stripping to obtain bisphenol A melt.

### (G) Prilling step

The bisphenol A melt obtained in the adduct decomposition step is fed to a tower head of a prilling tower and is sprayed through a number of holes provided in a nozzle plate placed at the tower head. The sprayed melt is cooled with a circulating gas flowing upward from a tower bottom of the prilling tower, and is taken out from the tower bottom in the form of granular solid referred to as prill, providing product bisphenol A.

### Examples

Hereinafter, the present invention will be further described in detail with reference to the following Examples, but it should be construed that the invention is in no way limited to those Examples.

### Reference Example

Through a fixed-bed reactor loaded with a cation-exchange resin ("DIAION(trade name) SK104H", manufactured by Mitsubishi Chemical Corporation) 20 mol% of which was partly neutralized with 2-mercapto ethylamine, phenol and acetone in a molar ratio of 10:1 were allowed to flow continuously at an LHSV of 3 hr⁻¹ and react at 75°C. The resulting reaction mixture liquid was introduced into a vacuum distillation column. After unreacted acetone, reaction product water in the reaction and the like were removed by vacuum distillation at a column bottom temperature of 170°C under a pressure of 67 kPa, further phenol was distilled out by vacuum distillation at a temperature of 130°C under a pressure of 14 kPa until bisphenol A was concentrated to 40 % by mass, and a bisphenol A/phenol concentrated liquid was obtained.
After that, water was added to the concentrated liquid, and the resulting mixture was kept at 45°C in a crystallizer under a reduced pressure of 2 kPa (15 Torr) for crystallization. The resultant slurry liquid was filtered with a horizontal belt filter. The resultant adduct crystals were washed twice at a washing ratio of 0.4 (with respect to wet adduct crystals) with phenol that was recovered by distillation. The resultant adduct crystals were subjected to re-crystallization.

Because water was present in an amount of 5 % by mass in the liquid (crystallization mother liquid) obtained by filtration with the horizontal belt filter, the water concentration in the mother liquid was adjusted at 0.5 % by mass using a distillation column. All of the mother liquid whose water concentration was adjusted as above was supplied to an isomerization reactor loaded with a cation exchange resin ("DIAION (registered trade name) SK104H", manufactured by Mitsubishi Chemical Corporation) at a reaction temperature of 75°C and an LHSV of 1 hr⁻¹.
Here, 80 % of the isomerized mother liquid was returned to the up-flow side of the vacuum distillation column so as to adjust the bisphenol A concentration at 40 % by mass, and the 20 % thereof were fed to the recovery step.
To the adduct crystals obtained by filtration with the horizontal belt filter, phenol recovered by evaporation and the mother liquid obtained in the re-crystallization step were added. The resultant mixture was heated at 90°C to prepare a liquid containing bisphenol A in an amount of 45 % by mass. After the liquid was filtered with a 10 µm filter, water was added again to the liquid, which was then subjected to re-crystallization at 5.33 kPa (40 Torr) and 50°C. The resultant slurry liquid was supplied to a two-stage pusher centrifuge (400G), washed with source phenol at a washing ratio (with respect to wet adduct crystals) of 0.4 so as to obtain wet adduct crystals. The resultant wet adduct was heated and melted at 130°C so as to remove phenol, and then the resultant was granulated in a spray prilling tower to obtain product bisphenol A prill.
After the resultant bisphenol A prill was heated at 175°C for 4 hours in the air, the prill exhibited a color of APHA 40 that was evaluated by eye-observation using APHA standard colors.

### Example 1

Except that before a mother liquid that was obtained in a solid-liquid separation step using a horizontal belt filter and had the water concentration adjusted at 0.5 % by mass in the mother liquid using a distillation column was isomerized, the mother liquid was treated in a free acid removing column loaded with a weak base anion exchange resin ("AMBERLIST" (registered trade mark) A21, manufactured by R&H Corporation) at a reaction temperature of 75°C and an LHSV of 1 hr⁻¹, an operation similar to Reference Example was performed so as to produce product bisphenol A. The resultant bisphenol A exhibited a color of APHA 15 and kept this quality over one year.

### Example 2

Except that after a mother liquid that was obtained in a solid-liquid separation step using a horizontal belt filter and had the water concentration adjusted at 0.5 % by mass in the mother liquid using a distillation column was isomerized, the mother liquid was treated in a free acid removing column loaded with a weak base anion exchange resin ("AMBERLIST" (registered trade name) A21, manufactured by R&H Corporation) at a reaction temperature of 75°C and an LHSV of 1 hr⁻¹, an operation similar to Reference Example was performed so as to produce product bisphenol A. The resultant bisphenol A exhibited a color of APHA 15 and kept this quality over one year.

### Example 3

Except that before a mother liquid that was obtained in a solid-liquid separation step using a horizontal belt filter and had the water concentration adjusted at 0.5 % by mass in the mother liquid using a distillation column was isomerized, the mother liquid was treated in a free acid removing column loaded with a strong acid cation exchange resin ("DIAIDN" (registered trade name) SK-104H, manufactured by Mitsubishi Chemical Corporation) and a weak base anion exchange resin ("AMBERLIST" (registered trade name) A21, manufactured by R&H Corporation) in a volume ratio of 1:1, at a reaction temperature of 75°C and an LHSV of 1 hr⁻¹, an operation similar to Reference Example was performed so as to produced product bisphenol A. The resultant bisphenol A exhibited a color of APHA 15 and kept this quality over one year.

### Example 4

Except that after a mother liquid that was obtained in a solid-liquid separation step using a horizontal belt filter and had the water concentration adjusted at 0.5 % by mass in the mother liquid using a distillation column was isomerized, the mother liquid was treated in a free acid removing column loaded with a strong acid cation exchange resin ("DIAION" (registered trade name) SK-104H, manufactured by Mitsubishi Chemical Corporation) and a weak base anion exchange resin ("AMBERLIST" (registered trade name) A21, manufactured by R&H Corporation) in a volume ratio of 1:1, at a reaction temperature of 75°C and an LHSV of 1 hr⁻¹, an operation similar to Reference Example was performed so as to produce product bisphenol A. The resultant bisphenol A exhibited a color of ALPHA 15 and kept this quality over one year.

### Comparative Example 1

Product bisphenol A was produced similarly to Example 1, except that the free acid removing column was not used. The resultant bisphenol A exhibited a color of APHA 40.

### Comparative Example 2

Product bisphenol A was produced similarly to Example 1, except that the free acid removing column was placed at the outlet of the concentration reactor. The resultant bisphenol A exhibited a color of APHA 35.

### Industrial Applicability

In an isomerization step that is one of the processes for producing bisphenol A, the present invention provides a process for producing high-quality bisphenol A with an excellent transparency (color) in particular by removing trace amount of free acid that is eluted off an acidic catalyst used in a reaction step and an isomerization acidic catalyst used in an isomerization step isomerizing the whole mother liquid obtained in a crystallization and solid-liquid separation step, and an apparatus for producing the bisphenol A.

## Claims

1. A process for producing bisphenol A, comprising a free-acid removing step in the following step (D) so as to remove free acid contained in a mother liquid or an isomerized liquid,
in a process for producing bisphenol A including: a condensation reaction step (A) reacting excess amount of phenol with acetone in the presence of an acidic catalyst, a concentration step (B) concentrating a reaction mixture obtained in the step (A), a crystallization and solid-liquid separation step (C) crystallizing an adduct of bisphenol A and phenol by cooling the concentrated reaction mixture obtained in the step (B) and separating into the adduct and a mother liquid, an isomerization step (D) isomerizing the whole of the mother liquid obtained in the step (C) with an isomerization catalyst and circulating the resultant isomerized liquid to the step (A) and/or step (B), an adduct decomposition step (F) preparing a bisphenol A melt by removing phenol from an adduct of bisphenol A and phenol obtained in the step (C), and a prilling step (G) preparing product prill by granulating the bisphenol A melt obtained in the step (F).

2. A process for producing bisphenol A, comprising a free-acid removing step in the following step (D) so as to remove free acid contained in a mother liquid or an isomerized liquid,
in a process for producing bisphenol A including; a condensation reaction step (A) reacting excess amount of phenol with acetone in the presence of an acidic catalyst, a concentration step (B) concentrating a reaction mixture obtained in the step (A), a crystallization and solid-liquid separation step (C) crystallizing an adduct of bisphenol A and phenol by cooling the concentrated reaction mixture obtained in the step (B) and separating into the adduct and a mother liquid, an isomerization step (D) isomerizing the whole of the mother liquid obtained in the step (C) with an isomerization catalyst and circulating part of the resultant isomerized liquid to the step (B), a recovery step (E) recovering an adduct of bisphenol A and phenol from a remaining portion of the isomerized liquid treated in the step (D), an adduct decomposition step (F) preparing a bisphenol A melt by removing phenol from an adduct of bisphenol A, and phenol obtained in the step (C), and a prilling step (G) preparing product prill by granulating the bisphenol A melt obtained in the step (F).

3. The process for producing bisphenol A according to claim 1 or 2, wherein the mother liquid before the isomerization is treated in the free-acid removing step so as to remove free acid.

4. The process for producing bisphenol A according to claim 1 or 2, wherein the isomerized liquid isomerized by passing the mother liquid through the isomerization catalyst is treated in the free-acid removing step so as to remove free acid.

5. The process for producing bisphenol A according to any of claims 1 to 4, wherein free acid is removed by using an anion exchange resin in the free-acid removing step.

6. The process for producing bisphenol A according to any of claims 1 to 4, wherein free acid is removed by using a mixture of an anion exchange resin and a cation exchange resin in the free-acid removing step.

7. The process for producing bisphenol A according to claim 5 or 6, wherein the anion exchange resin is a weak base anion exchange resin.

8. The process for producing bisphenol A according to claim 6, wherein the cation exchange resin is a strong acid cation exchange resin.

9. The process for producing bisphenol A according to any of claims 1 to 8, wherein free acid is removed in the presence of 0.01 to 1 % by mass of water in the free-acid removing step.

10. An apparatus for producing bisphenol A equipped with a free-acid removal measures to remove free acid contained in a mother liquid or an isomerized liquid in the isomerization step (D) in the process for producing Bisphenol A according to claim 1 or 2.
